# EUROPÄISCHE PATENTANMELDUNG

(11) **EP 2 965 779 A1**
(43) Veröffentlichungstag der Anmeldung: **13.01.2016**
(21) Anmeldenummer: 14176018.1
(22) Anmeldetag: 07.07.2014
(51) Int. Cl.: A61M 37/00

(54) **Applikationsmodul für ein Handgerät zum wiederholten Applizieren eines Applikationselementes auf eine menschliche oder eine tierische Haut und Handgerät**

(71) Anmelder: MT.DERM GmbH, 14167 Berlin (DE)
(72) Erfinder: Scherkowski, Dirk, 12489 Berlin (DE)
(74) Vertreter: Bittner, Thomas L.

(57) **Zusammenfassung**

Die Anmeldung betrifft ein Applikationsmodul (1) für ein Handgerät zum wiederholten Applizieren eines Applikationselementes (5) auf eine menschliche oder eine tierische Haut, mit einem Gehäuse (2), einer Applikationseinrichtung (4), die in dem Gehäuse angeordnet ist und eine an einer Applikationselementaufnahme (6) angeordnetes Applikationselement aufweist, wobei die Applikationselementaufnahme mit dem Applikationselement zwischen einer ausgefahrenen und einer eingefahrenen Stellung verlagerbar ist, einer Kopplungseinrichtung (7), die eingerichtet ist, funktionell an ein Antriebsmodul zu koppeln, so dass eine vom Antriebsmodul bereitgestellte Antriebskraft abgreifbar und zumindest für einen Antrieb der Applikationsrichtung bereitgestellt ist, einem an dem Gehäuse gebildeten Applikator (9), der im Betrieb in einem zu behandelnden Hautbereich zur Auflage kommt, einer Applikationselementkammer (8), in welcher sich das Applikationselement im Betrieb vor und zurückbewegt und die eine einem distalen Ende des Applikationselements zugeordnete Applikationselementöffnung aufweist, welche die Applikationselementkammer zum Applikator hin öffnet oder im Bereich des Applikators angeordnet ist, und einer Ansaugkammer (10), die getrennt von der Applikationselementkammer gebildet ist und einen Ansauganschluss (13) aufweist.

## Beschreibung

Die Erfindung betrifft ein Applikationsmodul für ein Handgerät zum wiederholten Applizieren eines Applikationselementes auf eine menschliche oder eine tierische Haut sowie ein Handgerät.

### Hintergrund

Derartige Handgeräte dienen dazu, ein Applikationselement wiederholt auf eine Haut zu applizieren. Zum Beispiel sind Vorrichtungen zum lokalen Aufstechen einer menschlichen oder einer tierischen Haut bekannt. Die Haut kann lokal behandelt werden, insbesondere aufgestochen werden.

Vom Bedienpersonal können Handgeräte, bei denen das Applikationselement als eine Stechelement ausgeführt ist, zum Aufbringen einer Farbe für eine Tätowierung (Tattoo) und / oder von permanentem Make-up im Bereich der Hautoberfläche verwendet werden. Aber auch das Einbringen kosmetischer oder medizinischer Wirkstoffe oder Substanzen über / in die Haut ist mit solchen Geräten möglich, indem die Haut lokal aufgestochen wird. Darüber hinaus können solche Geräte verwendet werden, ohne dass irgendeine Substanz eingebracht wird, zum Beispiel zur Hautstimulierung.

Ein Handgerät zum lokalen Aufstechen einer Haut ist beispielsweise aus der Druckschrift DE 299 19 199 U1 bekannt. Das bekannte Handgerät weist ein Griffstück, eine Antriebseinrichtung und ein Applikationselement auf, das mit Hilfe der Antriebseinrichtung im Betrieb relativ zu einer Nadeldüse vor und zurück bewegt wird, wobei mindestens zwei lösbar miteinander verbundene Module vorgesehen sind, und das eine der beiden Module als wieder verwendbares Grundmodul mit integrierter Antriebseinrichtung ausgebildet ist. Das andere der beiden Module ist ein sterilisiertes Einwegmodul, in das bei dem bekannten Handgerät alle durch die Köperflüssigkeiten eines Kunden kontaminierbaren Bestandteile integriert sind. Auf diese Weise wird das Handgerät in Form von zwei Modulen zur Verfügung gestellt, von denen eines, nämlich das Einwegmodul, nach der Benutzung ausgetauscht werden kann, während das andere Modul, welches die Antriebseinrichtung umfasst, wieder verwendet wird. Mit Hilfe des Einwegmoduls werden die hygienischen Bedingungen beim Aufbringen einer Tätowierung und / oder von permanentem Make-up verbessert, da alle Teile ausgetauscht werden, die potentiell durch die bei der Behandlung austretende Körperflüssigkeit des Kunden kontaminiert werden können. Es wird so vermieden, dass das gesamte Handgerät ausgetauscht werden muss.

Aus dem Dokument EP 1 495 782 A1 ist ein Antriebsmodul für eine Vorrichtung zum lokalen Aufstechen einer menschlichen oder einer tierischen Haut bekannt, bei dem eine Antriebseinrichtung, mit der eine Antriebsbewegung erzeugbar ist, und ein an die Antriebseinrichtung gekoppelter Wandlungsmechanismus vorgesehen sind, mit dem die Antriebsdrehbewegung in eine auf eine die Haut lokal aufstechende Applikationseinrichtung einkoppelbare Vorwärts- / Rückwärtsbewegung umgewandelt wird, so dass eine repetierende Bewegung eines Stechmittels ermöglicht ist.

### Zusammenfassung

Aufgabe der Erfindung ist es, ein Applikationsmodul für ein Handgerät zum wiederholten Applizieren eines Applikationselementes auf eine menschliche oder eine tierische Haut sowie ein Handgerät anzugeben, die ein verbessertes Applizieren auf die zu behandelnde Haut unterstützen.

Zur Lösung der Aufgabe sind ein Applikationsmodul für ein Handgerät zum wiederholten Applizieren eines Applikationselementes auf eine menschliche oder eine tierische Haut nach dem unabhängigen Anspruch 1 sowie ein Handgerät nach dem unabhängigen Anspruch 15 geschaffen. Vorteilhafte Ausgestaltungen sind Gegenstand von abhängigen Unteransprüchen.

Nach einem Aspekt ist ein Applikationsmodul für ein Handgerät zum wiederholten Applizieren eines Applikationselementes auf eine menschliche oder eine tierische Haut geschaffen, welches ein Gehäuse sowie eine Applikationseinrichtung aufweist, die in dem Gehäuse angeordnet ist und ein an einer Applikationsmittelaufnahme angeordnetes oder aufgenommenes Applikationselement aufweist, wobei die Applikationsmittelaufnahme mit dem Applikationselement zwischen einer ausgefahrenen und einer eingefahrenen Stellung verlagerbar ist. Im Betrieb kann hiermit ein distales Ende des Applikationselements in Kontakt mit der Haut gebracht und wieder von dieser weg bewegt werden, sei es die Haut aufstechend oder nicht aufstechend, zum Beispiel zu Massagezwecken. Das Applikationsmodul verfügt über eine Kopplungseinrichtung, die eingerichtet ist, funktionell an ein Antriebsmodul des Handgerätes zu koppeln, sei es direkt oder über ein oder mehrere zwischengeschaltete Kopplungselemente, so dass eine vom Antriebsmodul bereitgestellte Antriebskraft abgreifbar und zumindest für einen Antrieb der Applikationseinrichtung im Applikationsmodul bereitgestellt ist. Es ist ein an dem Gehäuse gebildeter Applikator vorgesehen, der im Betrieb in einem zu behandelnden Hautbereich zur Auflage kommt. Bei Verwendung des Applikationsmoduls am Handgerät kommt der Applikator in dem Hautbereich zur Auflage, in welchem die Haut wiederholt aufgestochen werden soll. Das Applikationselement bewegt sich im Betrieb in einer Applikationselementkammer einmalig oder wiederholt (repetierend) vor und zurück. Die Applikationselementkammer weist eine dem distalen Ende des Applikationselements zugeordnete Applikationselementöffnung auf, welche die Applikationselementkammer zum Applikator hin öffnet oder im Bereich des Applikators angeordnet ist. Das Applikationsmodul weist weiterhin eine Ansaugkammer auf, die getrennt von der Applikationselementkammer ist. An der Ansaugkammer ist ein Ansauganschluss gebildet, über den eine pneumatische Saugkraft in die Ansaugkammer einleitbar ist. Die Ansaugkammer steht mit einer Ansaugöffnung in Verbindung, die im Bereich des Applikators angeordnet ist. Im Betrieb wird die Ansaugöffnung, die mit einem oder mehreren Durchbrüchen (Teilöffnungen) gebildet sein kann, mit dem Applikator auf die Haut aufgesetzt, so dass ein der Ansaugöffnung gegenüberliegender Hautabschnitt angesaugt wird und wahlweise auch ein Stück in die Ansaugöffnung hineingezogen werden kann, wenn die pneumatische Saugkraft über den Ansauganschluss an die Ansaugkammer angelegt ist.

Nach einem weiteren Aspekt ist ein Handgerät zum wiederholten Applizieren eines Applikationselementes auf eine menschliche oder eine tierische Haut mit einem Antriebsmodul geschaffen, welches eingerichtet ist, eine Antriebskraft bereitzustellen. Das Handgerät weist weiterhin ein Applikationsmodul der vorgenannten Art auf, welches funktionell an das Antriebsmodul koppelt, sei es lösbar oder nicht lösbar, so dass die vom Antriebsmodul bereitgestellte Antriebskraft abgreifbar und im Applikationsmodul zumindest für einen Antrieb einer Applikationseinrichtung bereitgestellt ist.

Der Herausstand des Stechmittels in Bezug auf die Applikationselementöffnung kann einstellbar sein.

Es kann ergänzend in einer Ausgestaltung vorgesehen sein, dass eine zusätzliche pneumatische Saugkraft über die Applikationselementkammer bereitgestellt ist, wobei die pneumatische Saugkraft zum Beispiel über einen Ansauganschluss der Applikationselementkammer hierin einleitbar ist. Es kann alternativ oder ergänzend vorgesehen sein, dass ein die Applikationselementkammer und die Ansaugkammer trennender Wandabschnitt ein oder mehrere Durchbrüche aufweist, über die die pneumatische Saugkraft, welche an der Ansaugkammer anliegt, auch in die Applikationselementkammer eingeleitet wird. In einer alternativen Ausgestaltung ist die Applikationselementkammer im Betrieb frei von einer Ansaugkraft. Das Ansaugen der Haut bei aufgesetztem Applikator kann dann ausschließlich mittels der Ansaugkammer erfolgen, die ihrerseits bei dieser oder anderen Ausführungsformen als ein Einzel-oder ein Mehrkammersystem ausgeführt sein kann. Unabhängig von der Ausgestaltung als Ein- oder Mehrkammersystem kann die Ansaugöffnung mit einem oder mehreren Saugdurchbrüchen gebildet sein.

Die Applikationselementkammer kann von beliebiger Form sein und ein oder mehrere Teilkammern aufweisen. Das Applikationselement kann im Betrieb wenigstens zeitweise ganz oder nur teilweise hierin angeordnet sein.

Ein Übertritt der Saugkraft von der Ansaugkammer auf die Applikationselementkammer kann insbesondere dann unterbunden sein, wenn der Applikator auf die Haut aufgesetzt ist.

Das Applikationselement kann zumindest im Betrieb abschnittsweise auch durch die Ansaugkammer verlaufen, wobei ein Wanddurchgang des Applikationselementes in die Ansaugkammer hinein gedichtet ausgeführt sein kann.

Mit Hilfe der im Betrieb an die Ansaugkammer angekoppelten pneumatischen Saugkraft wird bei aufgesetztem Applikator die Haut festgehalten für das Einstechen des Stechmittels in die Haut. Hierbei kann vorgesehen sein, den eingekoppelten Saugdruck in Abhängigkeit von der Stellung des Stechmittels (eingefahren / ausgefahren) zu steuern. Beispielsweise kann die Saugkraft gemindert oder ganz abgeschaltet werden, wenn sich das Applikationselement in der eingefahrenen Stellung befindet und / oder sich aus der ausgefahrenen in die eingefahrene Stellung bewegt. Auch ein Nachlassen oder Abschalten einer zunächst anhaltenden Saugkraft nach einer vorbestimmten Anzahl von Stechbewegungen kann vorgesehen sein.

Das Applikationsmodul kann eingerichtet sein, bei wiederholten repetierenden Applikationsvorgängen, zum Beispiel Stechvorgängen, mit einer Wiederholfrequenz zwischen etwa 5 und etwa 500 Hz (Hübe pro Sekunde) betrieben zu werden, vorzugsweise zwischen etwa 50 und etwa 250 Hz. Das Antriebsmodul ist dann eingerichtet, eine entsprechende Antriebsbewegung bereitzustellen.

Die beim möglichen Hineinziehen der Haut in den Applikator auftretende Vergrößerung der Hautoberfläche kann eine Vorspannung der Haut und damit eine Verminderung des als Umbrella-Effekt bekannten Hautausweichens zum Beispiel bei Nadelbehandlungen bewirken, insbesondere im Bereich der vorderen Öffnung der Applikationselementmittelkammer. Eine weitere Steigerung der Hautvorspannung kann bei anhaltendem Saugdruck durch das Abheben der Applikatorfläche vom ursprünglichen Hautniveau erzielt werden, insbesondere, da sich wegen des leichten seitlichen Gleitens des Applikators bzw. der Applikatorfläche auf der Hautoberfläche und umgekehrt automatisch ein Ausgleich der Hautvorspannung im gesamten angehobenen und eingezogenen Hautbereich ergeben kann, wodurch ein trommelfellartiges Spannen der Haut vor der Mündung der Applikationselementkammer ausbildbar ist, was schließlich zu einem gleichmäßigeren Behandlungsergebnis führt und das Spannen mit zusätzlicher Fingerkraft unnötig macht, einer Ursache für eine ungewünschte Hämatombildung.

Das Applikationselement kann bei der Ausführung als Stechelement eine oder mehrere Einzelnadeln, eine oder mehrere Nadelgruppe und / oder eine oder mehrere Nadelplatte aufweisen. Letztere ist als solche zum Beispiel im Dokument EP 2 462 979 A1 offenbart. Das Applikationselement kann betreffend diese Ausgestaltungen auch übergreifend als Stechnadel bezeichnet werden, die an einer Nadelaufnahme angeordnet ist.

In einer Ausführungsform kann der Radius der Nadel oder der Nadeln so gewählt sein, dass bei einer Hautbehandlung die Hautstimulation durch eine mechanische Beanspruchung im Vordergrund steht.

Es kann vorgesehen sein, dass die Applikationselementöffnung und die Ansaugöffnung benachbart zueinander im Bereich einer Applikationsfläche angeordnet sind, die an einem distalen Ende des Applikators gebildet ist. Die Applikationsfläche kann sich zwischen vorderseitigen Stirnflächen des Applikators aufspannen. In einer Ausgestaltung ist die Ansaugöffnung in der Applikationsfläche angeordnet, wohingegen die Applikationselementöffnung hierzu zurückversetzt sein kann.

Bei einer Ausgestaltung kann vorgesehen sein, dass die Ansaugkammer wenigstens zum Teil von einem trompetenförmigen Wandabschnitt umgeben ist, welcher sich wenigstens teilweise auf einer Rückseite von der Ansaugöffnung weg erstreckt. Die trompetenförmige Ausgestaltung der Wand kann sich entlang einer Außenwand des Applikators umlaufend, durchgehend oder unterbrochen, erstrecken. Ist die Ansaugöffnung mit mehreren Ansaugdurchbrüchen gebildet, können die ganz oder teilweise von der trompetenförmigen Wand umgeben sein.

Es kann vorgesehen sein, dass sich die Ansaugkammer in einem der Ansaugöffnung gegenüberliegenden Kammerabschnitt im Querschnitt verjüngt. Der Verjüngungsabschnitt kann sich von der Ansaugöffnung ausgehend bis hin zum Ansauganschluss erstrecken oder auf einem Teil dieses Abstandes. Der Ansaugkammer zugewandte Wandabschnitte, zum Beispiel im Bereich des Verjüngungsabschnitts, können eben und / oder gewölbt ausgeführt sein, beispielsweise zum Innenraum der Ansaugkammer hin gewölbt.

Die Ansaugöffnung kann wenigstens teilweise von einem oder mehreren Wandabschnitten umgeben sein, die aus einem flexiblen Material bestehen. Das flexible Material kann elastisch nachgebend sein. Zum Beispiel kann ein Gummi-, ein Silikon- oder ein anderes Kunststoffmaterial verwendet werden.

Die Applikationselementkammer kann eine seitliche Öffnung aufweisen. Die seitliche Öffnung kann mit einem oder mehreren Durchbrüchen gebildet sein, sei es mit Rund- und / oder Schlitzform. Eine Öffnung kann sich von der Applikationsfläche ausgehend erstrecken.

Die Ansaugkammer kann die Applikationselementkammer mehrseitig umgeben. Bei dieser oder anderen Ausführungsformen kann vorgesehen sein, dass die Ansaugöffnung die Applikationselementöffnung ganz oder teilweise umgibt, oder umgekehrt. In einer Ausführung kann die Applikationselementöffnung längsgestreckt sein, wobei sich auf den beiden gegenüberliegenden Seiten der Applikationselementöffnung Ansaugdurchbrüche der Ansaugöffnung erstrecken. Auch kann in einer Ausführung eine Ansaugkammer innenliegend angeordnet sein, wobei außenliegend ein oder mehrere Ansaugöffnungen gebildet sind.

Eine Weiterbildung kann vorsehen, dass die Kopplungseinrichtung eingerichtet ist, eine mechanische, eine magnetische, eine elektrische und / oder eine pneumatische Antriebskraft zumindest für einen Antrieb der Applikationseinrichtung in dem Applikationsmodul bereitzustellen. Über die Kopplungseinrichtung können ein oder mehrere Antriebskräfte für die Funktionskomponenten in das Applikationsmodul eingekoppelt werden. Zum Beispiel kann eine mechanische Antriebskraft über ein Kupplungsgestänge übertragen werden. Alternativ oder ergänzend kann vorgesehen sein, eine pneumatische Antriebskraft über eine Fluidleitung einzukoppeln, sei es einen Überdruck und / oder ein Unterdruck, zum Beispiel eine pneumatische Antriebskraft. Ergänzend oder alternativ kann eine elektromagnetische Kopplung vorgesehen sein, zum Beispiel mit Hilfe eines oder mehrerer einander zugeordneter Magnete, die elektrische Magnete und / oder Dauermagnete umfassen können. Werden zum Beispiel eine lineare Antriebsbewegung sowie eine drehende Antriebsbewegung vom Antriebsmodul auf das Applikationsmodul übertragen, so kann im Applikationsmodul ein Bauteil zur Bewegungsentkopplung vorgesehen sein, derart, dass zum Beispiel die drehende Antriebsbewegung zum Antreiben einer Vakuumpumpe im Applikationsmodul und die lineare Antriebsbewegung zum Verlagern der Applikationsmittelaufnahme verwendet werden. Aber auch die lineare Antriebsbewegung kann zum Erzeugen der pneumatischen Saugkraft genutzt werden, beispielsweise dadurch, dass hiermit eine Vakuumpumpe angetrieben wird.

Die Kopplungseinrichtung kann eingerichtet sein, eine drehende und / oder eine geradlinige Antriebsbewegung abzugreifen. Zum Beispiel kann die mechanische Antriebsbewegung über ein Pleuelelement oder ein Gestänge von dem Antriebsmodul auf das Applikationsmodul übertragen werden. Bei dieser oder anderen Ausführungsformen kann in dem Antriebsmodul eine Antriebskraft zum Beispiel mit Hilfe eines Elektromotors bereitgestellt werden. Die Drehung einer Antriebswelle kann noch im Antriebsmodul oder dann im Applikationsmodul in eine Linearbewegung gewandelt werden, wozu ein Wandlungsmechanismus zum Einsatz kommt, wie diese als solche in verschiedenen Ausführungsformen bekannt sind.

Eine Ausführungsform sieht vor, dass die Kopplungseinrichtung eingerichtet ist, die vom Antriebsmodul bereitgestellte Antriebskraft kontaktlos abzugreifen. Ein kontaktloser Abgriff kann beispielsweise unter Verwendung einer Magnetkupplung mit einander zugeordneten Magneten außerhalb und innerhalb des Gehäuses des Applikationsmoduls ausgeführt werden. Das Gehäuse kann bei dieser oder anderen Ausgestaltungen zumindest im Bereich des Abgriffs der Antriebskraft als geschlossenes Gehäuse gebildet sein. In dieser oder anderen Ausführungsformen kann die Übertragung der Antriebsbewegung (Antriebskraft) in das Applikationsmodul mit Hilfe einer Magnetkupplung als Linear- oder als Drehbewegung erfolgen. Hierzu können im Applikationsmodul ein oder mehrere Magnete drehbar oder linear verlagerbar angeordnet sein, um eine entsprechende Bewegung von zugeordneten Magnetelementen außerhalb des Gehäuses des Applikationsmoduls nachzufolgen.

Die Kopplungseinrichtung kann mit einem Wandlungsmechanismus verbunden sein, welcher im Gehäuse angeordnet und eingerichtet ist, eine Antriebsbewegungsart in eine andere und auf die Applikationseinrichtung eingekoppelte Antriebsbewegungsart zu wandeln, welche von der Antriebsbewegungsart verschieden ist. Zum Beispiel kann mit dem Wandlungsmechanismus eine Drehbewegung in eine geradlinige oder lineare Bewegung gewandelt werden. Auch eine umgekehrte Wandlung der Antriebsbewegungsart kann vorgesehen sein. Wandlungsmechanismen als solche, insbesondere in einer Ausführung für Handgeräte zum wiederholten Aufstechen einer Haut, sind in verschiedenen Ausführungsformen bekannt. Zum Beispiel ist eine Kombination von Taumelscheibe und Stößel bekannt, mit der eine drehende Antriebsbewegung in eine lineare Bewegung zum Antreiben der Applikationseinrichtung wandelbar ist. In dem Gehäuse kann eine Pumpeinrichtung angeordnet sein, die mit dem Ansauganschluss der Ansaugkammer in Fluidverbindung steht, derart, dass ein von der Pumpeinrichtung erzeugter Ansaugdruck auf die Ansaugkammer gegeben werden kann. Die Pumpeinrichtung in dem Gehäuse kann als eine Vakuumpumpe ausgeführt sein. Bei der Pumpeinrichtung kann es sich um eine Kolbenpumpe handeln, wobei eine Luft fördernde Kolbenbewegung aufgrund einer mechanischen, einer magnetischen oder einer pneumatischen Antriebskraft ausgeführt werden kann. Alternativ oder ergänzend zum Erzeugen einer Saugkraft mit Hilfe der Pumpeinrichtung in dem Gehäuse kann das Einleiten einer Saugkraft, zum Beispiel Unterdruck, von außerhalb des Applikationsmoduls von einer externen Quelle vorgesehen sein. Hierzu kann das Gehäuse einen pneumatischen Anschluss aufweisen, über den ein Über- und / oder ein Unterdruck einleitbar ist. Der pneumatische Anschluss kann direkt oder über Verbindungselemente, beispielsweise ein oder mehrere Ventile, mit dem Ansauganschluss der Ansaugkammer in Fluidverbindung stehen.

Eine Weiterbildung kann vorsehen, dass in dem Gehäuse ein Fluidreservoir gebildet ist, welches eingerichtet ist, eine einzubringende Substanz aufzunehmen. Das Fluidreservoir kann zum Beispiel den Innenraum des Gehäuses erfassen, soweit dieser nicht von anderen Funktionskomponenten ausgefüllt ist. Es kann vorgesehen sein, dass hierbei das aufgenommene Fluid (Substanz) andere im Innenraum angeordnete Funktionskomponenten umgibt. Auch ein gegenüber dem Innenraum abgegrenztes Fluidreservoir kann vorgesehen sein. Über eine Fluidverbindung gelangt das aufgenommen Fluid vom Reservoir zum Applikationselement und kann auf dessen Oberfläche aufgrund des Vor- und Zurückbewegens ausgebracht werden. Es kann vorgesehen sein, das Fluidreservoir mit einem Druck zu beaufschlagen, welcher das Ausbringen des Fluids im Betrieb unterstützt. Bei dem Fluid kann es sich beispielsweise um einen Farbstoff zum Herstellen eines Tattoos oder von permanentem Make-up handeln. Aber auch medizinische und / oder kosmetische Substanzen können zum Einbringen in die Haut und / oder zum Aufbringen auf die Hautoberfläche vorgesehen sein. Ist das Applikationselement mit einer Hohlnadel gebildet, kann die Substanz ergänzend oder alternativ durch den inneren Hohlraum der Hohlnadel ausgebracht werden, sei es ausschließlich oder ergänzend hierdurch. Es kann vorgesehen sein, mittels der über die Ansaugkammer bereitgestellten Saugwirkung nicht in die Haut gelangte Reste des ausgebrachten Fluids wieder in dem Applikationsmodul aufzunehmen (einzusaugen) und dem Fluidreservoir zuzuführen. Hierbei kann sich bei einem schäumbaren Fluid ein stabiler Schaum bilden, der das Applikationselement und / oder die Haut benetzt und hierbei weiteres übermäßiges oder versehentliches Austreten des Fluids eindämmt bzw. verhindert. Bei entsprechend angepasstem Schäumungsverhalten des Fluids können so auch die Dosierstärke und der Wirkstoffverbrauch kontrolliert werden.

Die Applikationseinrichtung kann eine Stecheinrichtung sein, die an der als Stechelementaufnahme ausgeführten Applikationselementaufnahme das als ein Stechelement gebildete Applikationselement aufweist. Am distalen Ende des Stechelements, welches mit einer oder mehreren Nadeln gebildet sein kann, ist eine Stechspitze angeordnet, die eingerichtet ist, die Haut aufzustechen. Der Stechspitze ist dann in der Stechmittelkammer eine Nadel- oder Stechmittelöffnung zugeordnet.

Alternativ kann die Applikationseinrichtung eine nicht-stechende Applikationseinrichtung sein, bei der an der Applikationselementaufnahme ein nicht-stechendes Applikationselement angeordnet ist, das eingerichtet ist, nicht-stechend auf die Haut angewendet zu werden, zum Beispiel zum Massieren derselben. Das distale Ende des Applikations- oder Behandlungselement ist dann frei von einer Stechspitze, sondern eher gerundet, zum Beispiel eine Ball- oder Kugelform aufweisend, oder mit einer flachen Frontfläche gebildet.

Es kann vorgesehen sein, dass die Applikationselementaufnahme ein Verkippen und / oder ein Verdrehen des Applikationselementes zulassend ausgeführt ist, insbesondere bei der Ausführung des Applikationselementes als nicht-stechendes Applikationselement. Im Betrieb kann es dann zum Beispiel zum Verkippen / Verdrehen des Applikationselements kommen, wenn dieses gegen die Haut gedrückt wird. Bei dem nicht-stechenden Applikationselement kann alternativ oder ergänzend zum Vor und Zurückbewegen vorgesehen sein, dieses im Betrieb zu drehen um eine Achse, die sich in Längsrichtung des Gehäuses des Applikationsmoduls erstreckt.

Der Applikator kann lösbar an dem Gehäuse angeordnet sein. Es kann ein Bausatz mit einem Applikationsmodul und mehreren verschiedenen Applikatoren vorgesehen sein. Ein solcher Artikel ermöglicht es dem Nutzer, das Applikationsmodul mittels Anbringen eines jeweils gewünschten Applikators für unterschiedliche Anwendungen einzurichten. Die Applikatoren können sich beispielsweise hinsichtlich ihrer Größe und / oder ihrer Formgestaltung unterscheiden, insbesondere bezüglich der Größe und / oder Form einer Applikationsfläche.

Mit einem Handgerät, welches Antriebsmodul und hieran koppelndes Applikationsmodul aufweist, ist ein Verfahren zum wiederholten Applizieren eines Applikationselementes auf eine menschliche oder eine tierische Haut ausführbar. Die Applikationsmittelaufnahme mit dem Applikationselement wird im Betrieb zwischen einer ausgefahrenen und einer eingefahrenen Stellung verlagert, derart dass ein distales Ende des Applikationselements in Kontakt mit der Haut gebracht und wieder von dieser weg bewegt wird, sei es die Haut aufstechend oder nicht aufstechend, zum Beispiel zu Massagezwecken. Es ist ein an dem Gehäuse des Applikationsmoduls gebildeter Applikator vorgesehen, der im Betrieb in einem zu behandelnden Hautbereich zur Auflage kommt. Bei Verwendung des Applikationsmoduls am Handgerät kommt der Applikator in dem Hautbereich zur Auflage, in welchem die Haut wiederholt bearbeitet werden soll, sei es diese aufstechend oder nicht. Das Applikationselement bewegt sich im Betrieb in einer Applikationselementkammer einmalig oder wiederholt (repetierend) vor und zurück. Die Applikationselementkammer weist eine dem distalen Ende des Applikationselements zugeordnete Applikationselementöffnung auf, welche die Applikationselementkammer zum Applikator hin öffnet oder im Bereich des Applikators angeordnet ist. Das Applikationsmodul weist weiterhin eine Ansaugkammer auf, die getrennt von der Applikationselementkammer ist. An der Ansaugkammer ist ein Ansauganschluss gebildet, über den eine pneumatische Saugkraft in die Ansaugkammer eingeleitet wird. Die Ansaugkammer steht mit einer Ansaugöffnung in Verbindung, die im Bereich des Applikators angeordnet ist. Im Betrieb wird die Ansaugöffnung mit dem Applikator auf die Haut aufgesetzt, so dass ein der Ansaugöffnung gegenüberliegender Hautabschnitt angesaugt wird und wahlweise auch ein Stück in die Ansaugöffnung hineingezogen werden kann, wenn die pneumatische Saugkraft über den Ansauganschluss an die Ansaugkammer angelegt ist. Zeitlich parallel zum Ansaugen der Haut wird das Applikationselement ausgefahren.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung eines Applikationsmoduls für ein Handgerät zum wiederholten Aufstechen einer menschlichen oder einer tierischen Haut im Querschnitt, bei dem eine lineare Antriebsbewegung eingekoppelt wird,
- Fig. 2: eine schematische Darstellung eines weiteren Applikationsmoduls im Querschnitt, bei dem eine lineare Antriebsbewegung eingekoppelt wird,
- Fig. 3: eine schematische Darstellung eines Applikationsmoduls im Querschnitt, bei dem neben einer linearen Antriebsbewegung ein Überdruck von außen am Applikationsmodul bereitgestellt wird,
- Fig. 4: eine schematische Darstellung eines anderen Applikationsmoduls im Querschnitt, bei dem neben einer linearen Antriebsbewegung ein Unterdruck von außen am Applikationsmodul bereitgestellt wird,
- Fig. 5: eine schematische Darstellung eines Applikationsmoduls im Querschnitt, bei dem mehrere Pneumatikanschlüsse vorgesehen sind,
- Fig. 6: eine schematische Darstellung eines Applikationsmoduls im Querschnitt, bei dem eine drehende Antriebsbewegung eingekoppelt wird,
- Fig. 7: eine schematische Darstellung eines Applikationsmoduls im Querschnitt, bei dem eine lineare Antriebsbewegung sowie eine drehende Antriebsbewegung eingekoppelt werden,
- Fig. 8: eine schematische Darstellung eines Applikationsmoduls im Querschnitt, bei dem über ein und dieselbe Kopplung sowohl eine lineare wie auch eine drehende Antriebsbewegung eingekoppelt und im Applikationsmodul entkoppelt werden,
- Fig. 9: eine schematische Darstellung eines Applikationsmoduls im Querschnitt, bei dem elektrische Antriebsenergie eingekoppelt wird,
- Fig. 10: eine schematische Darstellung eines anderen Applikationsmoduls im Querschnitt, bei dem elektrische Antriebsenergie eingekoppelt wird,
- Fig. 11: eine schematische Darstellung eines Applikationsmoduls im Querschnitt, bei dem eine lineare Antriebsbewegung sowie über einen Pneumatikanschluss von außen ein Unterdruck eingekoppelt werden,
- Fig. 12: eine schematische Darstellung eines Applikationsmoduls im Querschnitt, bei dem eine drehende Antriebsbewegung sowie über einen Pneumatikanschluss von außen ein Unterdruck eingekoppelt werden,
- Fig. 13: eine schematische Darstellung einer Kopplungseinrichtung zum kontaktlosen Abgreifen einer rotierenden Antriebsbewegung mittels einer Magnetkupplung,
- Fig. 14: eine schematische Darstellung eines vorderen Abschnitts eines Applikators im Querschnitt zum Erläutern des Ansaugens einer Hautoberschicht,
- Fig. 15: eine schematische Darstellung eines vorderen Abschnitts eines Applikators im Querschnitt, wobei ein Wandungsabschnitt aus flexiblem Material ist,
- Fig. 16: eine schematische Darstellung einer Applikationsfläche eines Applikators in Draufsicht, wobei die Applikationsfläche nierenförmig ausgeformt ist,
- Fig. 17: eine schematische Darstellung zum Auflegen eines Applikators im Bereich einer Augenpartie,
- Fig. 18: eine schematische Darstellung zum Auflegen eines Applikators im Bereich einer Augenpartie, wobei eine zu behandelnde Fläche schraffiert ist,
- Fig. 19: eine schematische Darstellung einer Applikationsfläche an einem Appliktor in Draufsicht und im Querschnitt,
- Fig. 20: eine schematische Darstellung einer anderen Applikationsfläche an einem Applikator in Draufsicht und im Querschnitt,
- Fig. 21: eine schematische Darstellung einer weiteren Applikationsfläche an einem Applikator in Draufsicht und im Querschnitt,
- Fig. 22: eine schematische Darstellung einer Applikationsfläche an einem Applikator mit seitlich angeordnetem Stechmittel,
- Fig. 23: eine schematische Darstellung einer anderen Applikationsfläche an einem Applikator mit ebenfalls seitlich angeordnetem Applikationselement und
- Fig. 24: eine schematische Darstellung einer weiteren Applikationsfläche an einem Applikator mit seitlich angeordnetem Stechmittel.

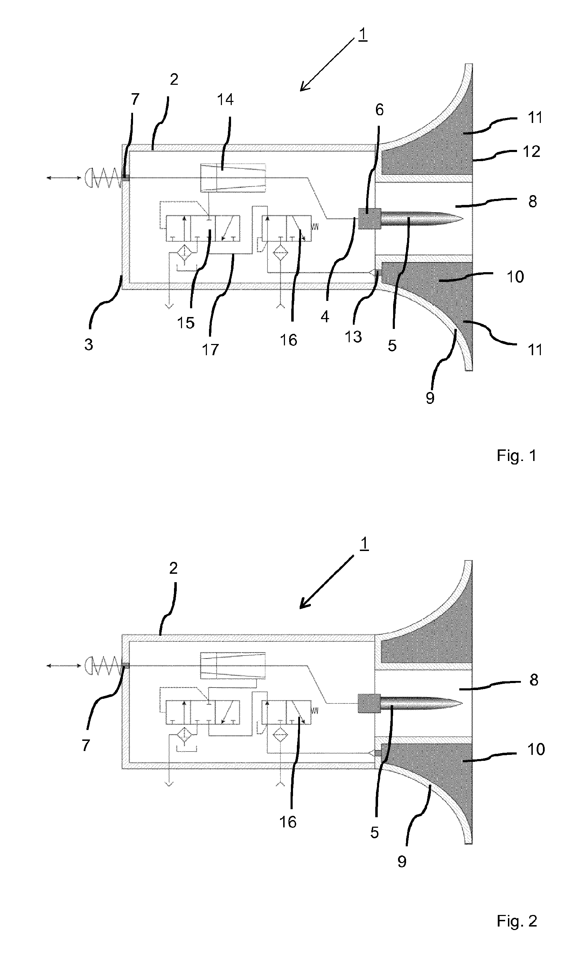

Fig. 1 zeigt eine schematische Darstellung eines Applikationsmoduls 1, welches in dem gezeigten Ausführungsbeispiel als ein Stechmodul ausgeführt ist für ein Handgerät zum wiederholten Aufstechen einer menschlichen oder einer tierischen Haut. Es ist ein Gehäuse 2 vorgesehen, bei dem eine rückseitige Fläche 3 einem Antriebsmodul (nicht dargestellt) des Handgerätes zugewandt sein kann, wenn das Applikationsmodul 1 funktionell an das Antriebsmodul des Handgerätes koppelt, sei es lösbar oder nicht lösbar. Eine lösbare Befestigung kann zum Beispiel mittels Schraub-, Klemm- und / oder Steckverbindung gebildet sein. Das Applikationsmodul 1 kann als Einwegmodul ausgeführt sein. Insbesondere das Gehäuse 2 kann aus einem Kunststoffmaterial bestehen, zum Beispiel als Spritzgussteil ausgeführt sein.

In dem Gehäuse 2 ist eine Applikationseinrichtung 4 mit einem Applikationselement 5 angeordnet, das an einer Applikationsmittelaufnahme 6 aufgenommen ist. Im Betrieb, also wenn das Applikationsmodul 1 an das Antriebsmodul des Handgerätes zum Aufstechen der Haut angeordnet ist, wird über eine Kopplungseinrichtung 7 eine vom Antriebsmodul bereitgestellte Antriebskraft, insbesondere als Antriebsbewegung, in das Applikationsmodul 1 eingekoppelt, um eine Vor- und Zurückbewegung der Applikationsmittelaufnahme 6 mit dem hieran angeordneten Applikationselement 5 auszuführen, so dass die Applikationsmittelaufnahme 6 mit dem Applikationselement 5 zwischen einer eingefahrenen und einer ausgefahrenen Stellung in Längsrichtung des Gehäuses 2 verlagert wird. Bei der in Fig. 1 gezeigten Ausführungsform, was auch für die Ausgestaltung in Fig. 2 gilt, wird über die Kopplungseinrichtung 7 eine Linearbewegung eingekoppelt, die auf die Applikationseinrichtung 4 übertragen wird.

Das Applikationselement 5 ist in einer Applikationselementkammer 8 angeordnet, die bei der gezeigten Ausführungsform in einem Applikator 9 gebildet ist. Die Applikationselementkammer 8 ist getrennt von einer Ansaugkammer 10, die sich gemäß den Fig. 1 und 2 bei den dargestellten Ausführungsformen beidseitig der Applikationselementkammer 8 erstreckt. Die Ansaugkammer 10 öffnet sich nach vorne über eine Ansaugöffnung 11, die ihrerseits in einer Applikationsfläche 12 auf der Vorderseite des Applikators 9 liegt.

Im Betrieb des Handgerätes wird der Applikator 9 im Bereich der Applikationsfläche 12 auf die zu behandelnde Haut aufgesetzt. Über einen Ansauganschluss 13 wird ein von einer Vakuumpumpe 14 und über eine Ventileinrichtung 15 sowie eine weitere Ventileinrichtung 16 geführter Saugdruck auf die Ansaugkammer 10 gegeben, so dass dort die Hautoberfläche angesaugt wird. Je nach Stellung der Ventileinrichtung 15 sowie der weiteren Ventileinrichtung 16 wird der Unterdruck an dem Ansauganschluss 13 angelegt oder nicht. Dieses erfolgt dann, wenn eine Fluidverbindung 17 von der Vakuumpumpe 14 zu dem Ansauganschluss 13 durchgeschaltet ist.

Bei der Ausführung des Applikationsmoduls 1 in Fig. 2 erfolgt eine Luftförderung bei der Rückstellbewegung der Applikationseinrichtung 4, wohingegen dies bei der Ausführungsform in Fig. 1 bei der Ausfahrbewegung erfolgt. Antriebsbewegung für die Applikationsmittelaufnahme 6 und Bewegung zur Förderung von Luft in der Vakuumpumpe 14 sind gekoppelte Bewegungen. Zum Beispiel koppeln ein oder mehrere Gestängeelemente, die ihrerseits mit der Applikationsmittelaufnahme 6 verbunden sind, an einen Kolben der Vakuumpumpe 14, so dass die Kolbenbewegung in Längsrichtung des Gehäuses 2 auf die Applikationsmittelaufnahme 6 eingekoppelt werden kann.

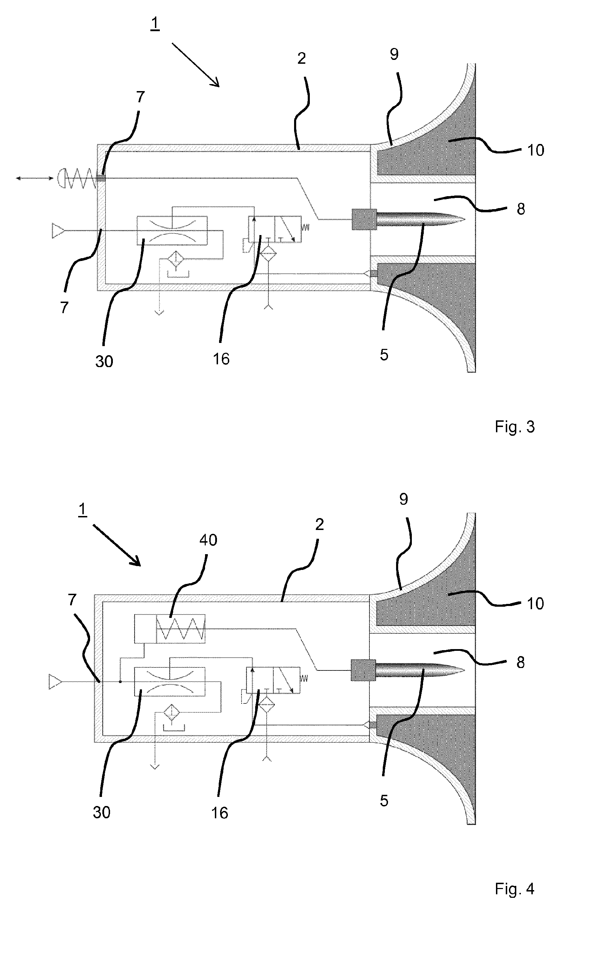

Bei den Fig. 3 bis 12 werden für gleiche Merkmale dieselben Bezugszeichen wie in den Fig. 1 und 2 verwendet.

Bei der Ausgestaltung des Applikationsmoduls in Fig. 3 wird, vergleichbar den Ausgestaltungen in den Fig. 1 und 2, eine lineare Antriebsbewegung über die Kopplungseinrichtung 7 ins Applikationsmodul 1 eingekoppelt. Zum Erzeugen des Unterdrucks für die Ansaugkammer 10 wird ein Injektor 30 mit Überdruck von einer äußeren Quelle (nicht dargestellt) versorgt, der beim Durchströmen des Pneumatikgases mittels des bekannten Venturi-Effekts einen Unterdruck bereitstellt. In dieser Hinsicht gleichartig ausgebildet ist die Ausführungsform in Fig. 4, wobei hier nun der von extern bereitgestellte, pneumatische Druck genutzt wird, um ein Bauteil 40 mit einem Pneumatikzylinder anzutreiben, so dass hierdurch die für die Applikationseinrichtung 4 notwendige Antriebsbewegung bereitgestellt wird.

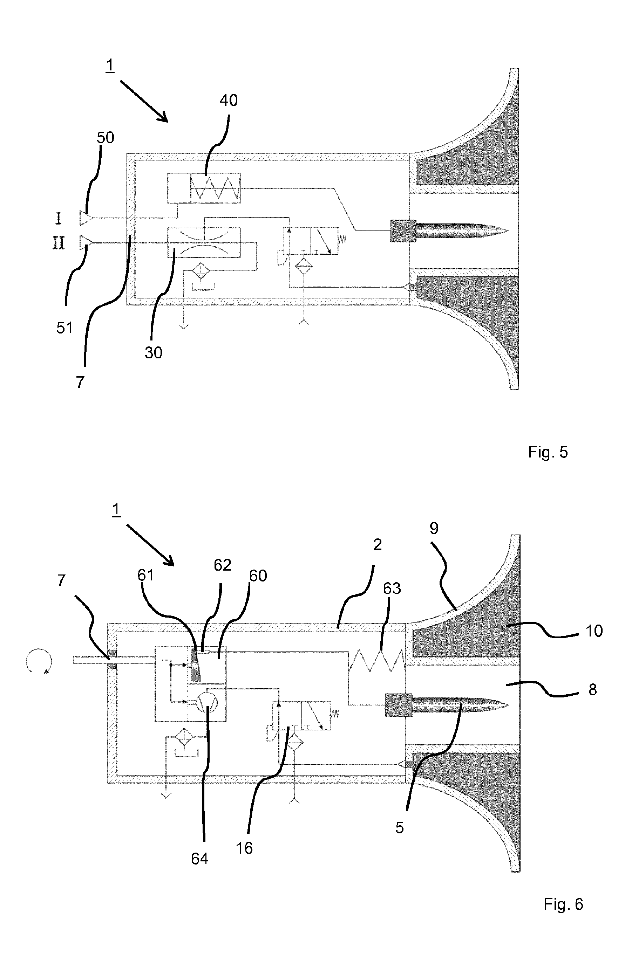

In ähnlicher Weise arbeitet die Ausführungsform des Applikationsmoduls 1 nach Fig. 5, wobei Injektor 30 und Bauteil 40 mit Pneumatikzylinder nun über getrennte Anschlüsse 50, 51 versorgt werden, bei dem es sich zum Beispiel um separate Druckluftanschlüsse handelt.

Bei den Ausgestaltungen des Applikationsmoduls 1 in den Fig. 6, 7 und 8 wird jeweils eine drehende Antriebsbewegung in das Applikationsmodul 1 eingekoppelt. Bei der Ausgestaltung in Fig. 6 treibt die drehende Antriebsbewegung einen Wandlungsmechanismus 60, mit dem die drehende Antriebsbewegung in eine lineare Antriebsbewegung in Längsrichtung des Gehäuses 2 umgewandelt wird, um die Applikationseinrichtung 4 anzutreiben. Der Wandlungsmechanismus 60 ist hierzu bei dem gezeigten Ausführungsbeispiel mit einer Taumelscheibe 61 und einem Stößel 62 gebildet. Eine Rückstellfeder 63 sorgt für die Rückstellbewegung der Applikationsmittelaufnahme 6 mit der Applikationselement 5. Weiterhin wird mittels der drehenden Antriebsbewegung eine Vakuumpumpe 64 betrieben.

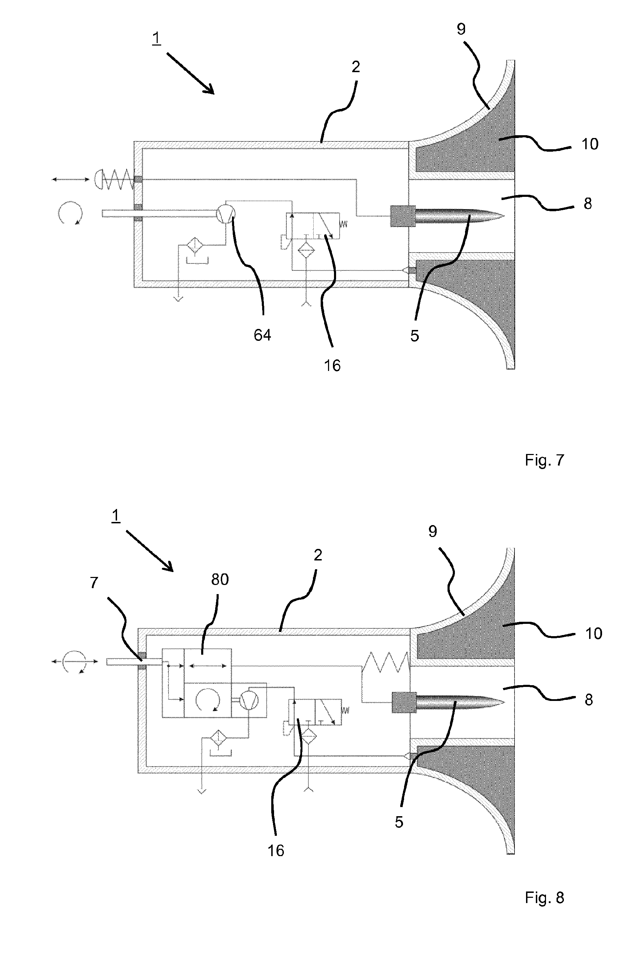

In Fig. 7 wird ergänzend zur drehenden Antriebsbewegung eine lineare Antriebsbewegung eingekoppelt, vergleichbar den Ausgestaltungen in den Fig. 1 bis 3. Der Wandlungsmechanismus 60 ist dann weggelassen.

Bei der Ausführungsform des Applikationsmoduls 1 in Fig. 8 werden drehende und lineare Antriebsbewegung gemeinsam in das Applikationsmodul 1 eingekoppelt, um die beiden Antriebsbewegungen dann mit Hilfe einer Entkopplungseinrichtung 80 im Gehäuse 2 zu entkoppeln. Beispielsweise kann die drehende von der linearen Antriebsbewegung eines antriebsseitigen Kopplungsteils im Applikationsmodul 1 entkoppelt werden, indem einerseits ein drehbar gelagertes Teil einer Vakuumpumpe, beispielsweise eines Flügelzellenverdichters, mit gemeinsamer Drehachse auf dem es durchgreifenden antriebsseitigen Kopplungsteil, welches in seiner äußeren prismatischen Kontur zur Drehmomentübertragung gestaltet ist, ortsfest drehend und dabei relativ längs gleitend koppelt, derart, dass hierbei lediglich die Drehbewegung übertragen wird. Andererseits kann die Entkopplung der linearen Antriebsbewegung mittels eines an die Stirnfläche und in der Drehachse des antriebsseitigen Kopplungsteils koppelnden, ausschließlich linear vor- und zurück beweglichen Stößels mit vorzugsweise verschwindender Berührfläche erfolgen, der mit der Applikationseinrichtung 4 verbunden ist.

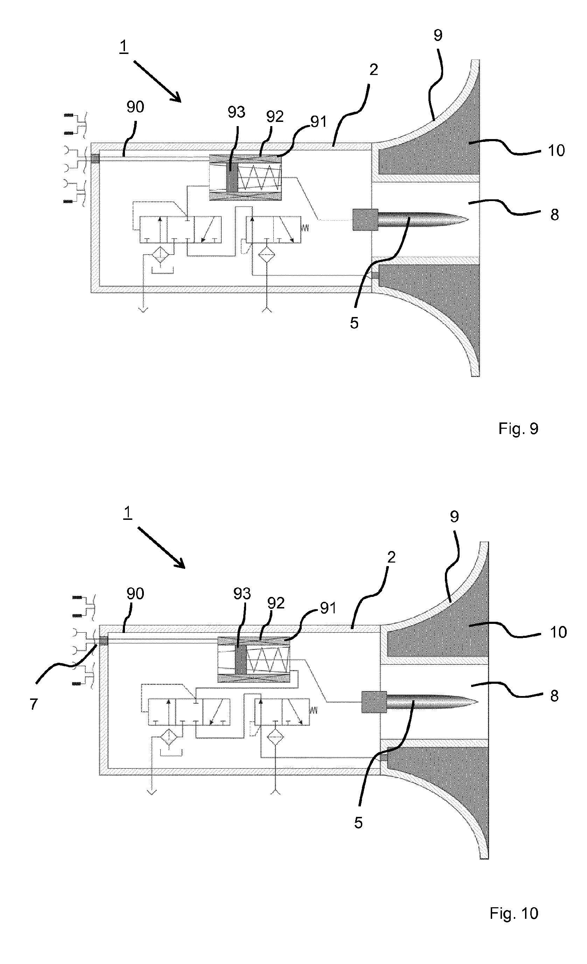

Die Fig. 9 und 10 zeigen schematische Darstellungen für Ausführungsformen des Applikationsmoduls 1, bei denen eine elektrische Antriebsenergie über eine Anschlussleitung 90 bereitgestellt wird, um hiermit eine Pumpeinrichtung 91 anzutreiben. Bei dieser ist im magnetischen Feld einer zumindest zeitweise von elektrischem Strom durchflossenen Spule 92 ein Pneumatikkolben mit Permanentmagnet und / oder Weicheisenkern 93 angeordnet, welcher mittels elektrischer Steuerung in Längsrichtung des Gehäuses 2 vor- und zurückbewegt wird, wodurch einerseits die lineare Antriebsbewegung für die Applikationseinrichtung 4 und andererseits die Vakuumerzeugung ausgeführt werden. Während bei der Ausführungsform in Fig. 9 Luft bei der Ausfahrbewegung der Applikationseinrichtung 4 gefördert wird, erfolgt dies bei der Ausgestaltung nach Fig. 10 bei der Rückstellbewegung.

Es kann alternativ vorgesehen sein, dass die Spule 92 statt im Applikationsmodul 1 außerhalb des Gehäuses 2 angeordnet ist, wahlweise getrennt vom Applikationsmodul 1, zum Beispiel in das Antriebsmodul (nicht dargestellt) integriert ist und auf das Applikationsmodul 1 so ein entsprechendes zeitlich veränderliches Magnetfeld kontaktlos wirken kann, welches einen wie oben beschriebenen Pneumatikkolben zu bewegen vermag. Zum Beispiel kann das Gehäuse 2 von einem Abschnitt des Antriebsmoduls wenigstens teilweise umgriffen werden, an dem die Spule angeordnet ist, so dass sich dies längs des Gehäuses 2 erstreckt, insbesondere seitlich des Pneumatikkolbens mit Permanentmagnet und / oder Weicheisenkern 93. Der Spulenkörper oder ein die Spule 92 einfassendes Gehäuse können auf der äußeren Oberfläche des Gehäuse 2 aufliegen.

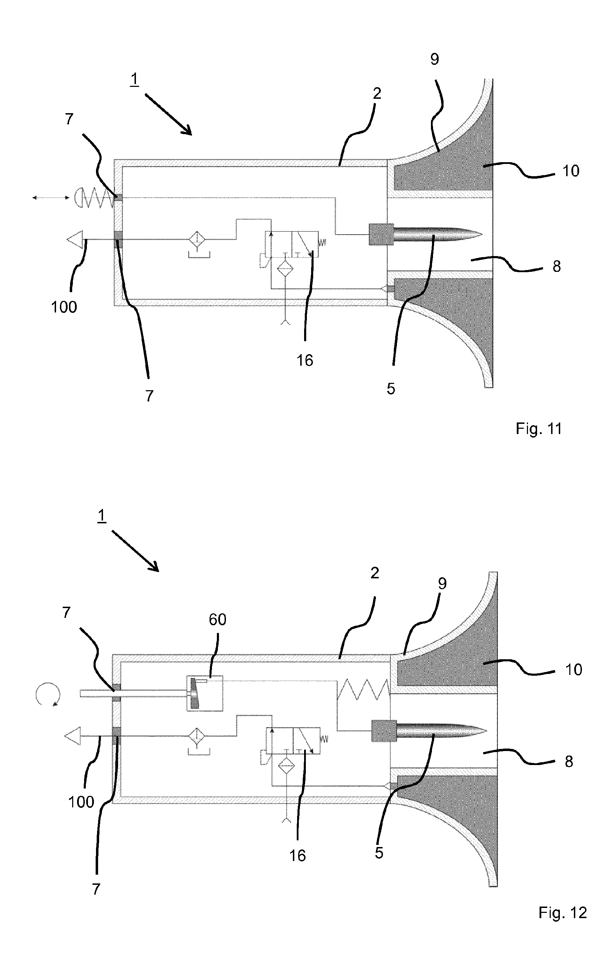

Die Ausführungsformen in den Fig. 11 und 12 des Applikationsmoduls 1 zeigen Anordnungen, bei denen eine lineare Antriebsbewegung (Fig. 11) oder eine drehende Antriebsbewegung (Fig. 12) eingekoppelt werden, wie dies oben bereits erläutert wurde. Ein externer Unterdruck wird über einen Pneumatikanschluss 100 angelegt und über die weitere Ventileinrichtung 16 betriebsabhängig an den Ansauganschluss 13 gegeben.

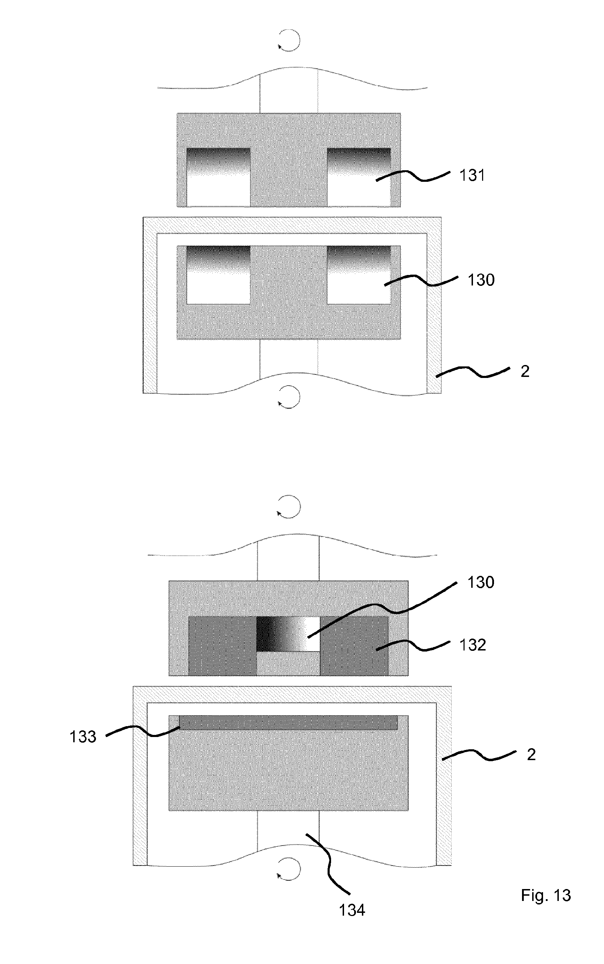

Die Fig. 13 zeigt schematische Darstellungen einer Kopplungseinrichtung zum kontaktlosen Abgreifen einer drehenden Antriebsbewegung mit Hilfe einer jeweiligen Magnetkupplung, bei der innerhalb und außerhalb des Gehäuses 2 einander zugeordnete Magnete 130, 131 angeordnet sind. Im Bereich zwischen den einander zugeordneten Magneten 130, 131 und benachbart hierzu ist das Gehäuse 2 als geschlossenes Gehäuse gebildet. In Fig. 13 ist in der unteren Darstellung eine Magnetkupplung dargestellt, die antriebsseitig mit einem Permanentmagnet 130 und einem ersten Teil 132 eines magnetischen Rückschlusssystems gebildet ist, wobei ein komplettierender Teil 133 dieses Systems sich auf dem drehbaren Kopplungsteil 134 im Applikationsmodul 1 befindet.

In analoger Weise zu den vorgenannten Ausgestaltungen, bei denen eine drehende Antriebsbewegung kontaktlos eingekoppelt wird, kann vorgesehen sein, eine lineare Antriebsbewegung kontaktlos über eine Magnetkopplung einzukoppeln. Hierbei können sich ein oder mehrere Magnete (nicht dargestellt) außerhalb des Gehäuses 2 in dessen Längsrichtung bewegen. Im Gehäuse 2 wird die Bewegung abgegriffen mittels zugeordneter Magnete.

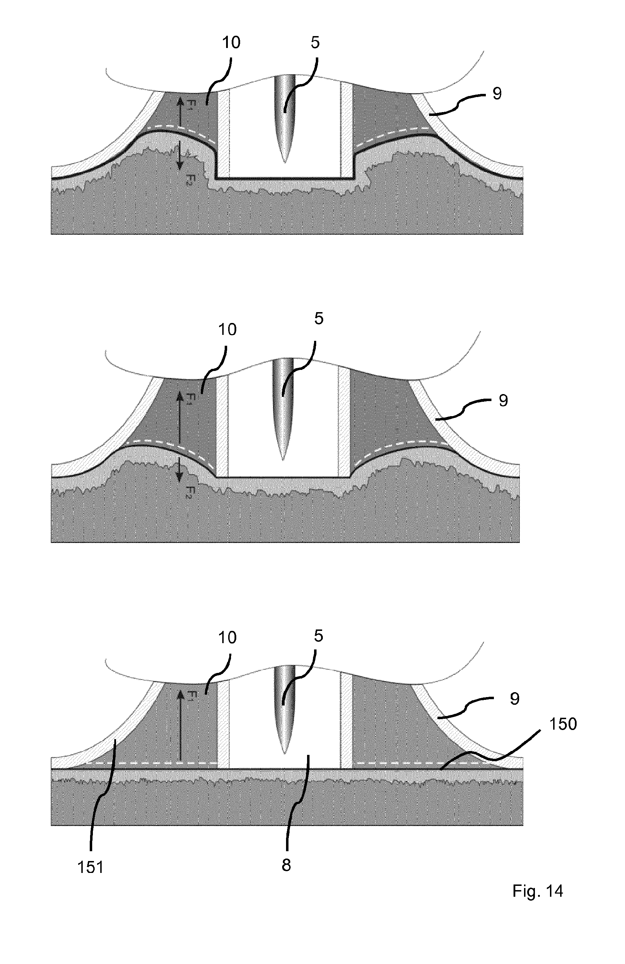

Fig. 14 zeigt eine schematische Darstellung eines vorderen Abschnitts des Applikators 9, wenn dieser auf eine Hautoberfläche 150 aufgelegt ist. Das Applikationselement 5 ist in einer eingefahrenen Stellung gezeigt. Mittels Anlegen des Ansaugdrucks über den Ansauganschluss 13 wird die Hautoberfläche in die Ansaugkammer 10 hineingezogen (vgl. mittlere Darstellung in Fig. 14), bis sich ein Kräftegleichgewicht (vgl. obere Darstellung in Fig. 14) eingestellt hat. Die Hautoberfläche 150 schmiegt sich hierbei an die nach innen zur Ansaugkammer 10 hin gewölbte Oberfläche 151 an.

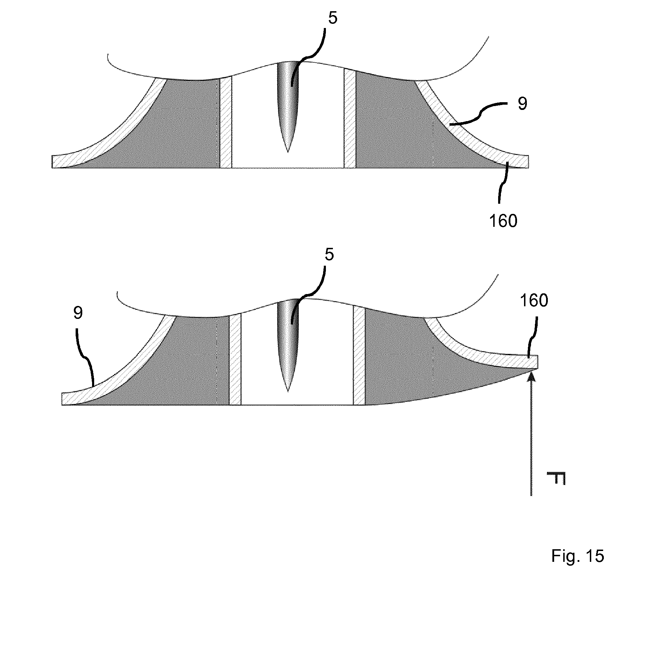

Fig. 15 zeigt eine Darstellung vergleichbar zu Fig. 14, wobei eine Wandung 160 des Applikators 9 aus einem elastisch nachgebenden Material besteht, so dass es beim Auflegen auf die zu behandelnde Hautoberfläche zum Umbiegen des Wandabschnitts 160 kommen kann, beispielsweise zum Ausgleich von Unebenheiten im Hautbereich oder von schrägem Aufsetzen des Applikationsmoduls 1 (vgl. untere Darstellung in Fig. 15).

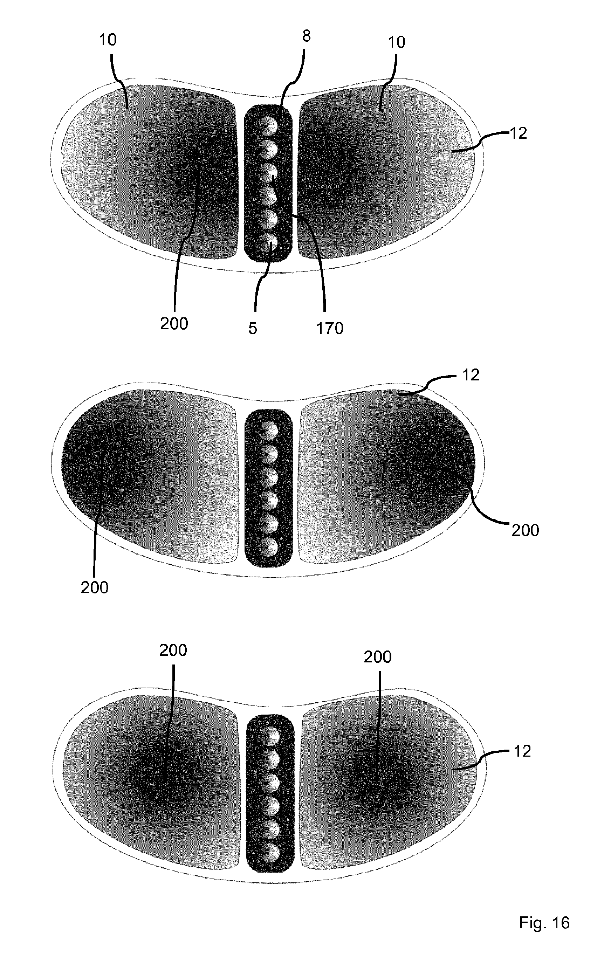

Fig. 16 zeigt Darstellungen der Applikationsfläche 12 für eine Ausführungsform eines Applikators 9, bei der das Applikationselement 5 mit einer Nadelgruppe 170 innerhalb der Applikationselementkammer 8 gebildet ist. Beidseitig der Applikationselementkammer 8 erstreckt sich die Ansaugkammer 10, die mit mehreren Ansaugöffnungen gebildet ist. Die Applikationsfläche 12 ist in der dargestellten Ausführungsform insgesamt nierenförmig ausgebildet.

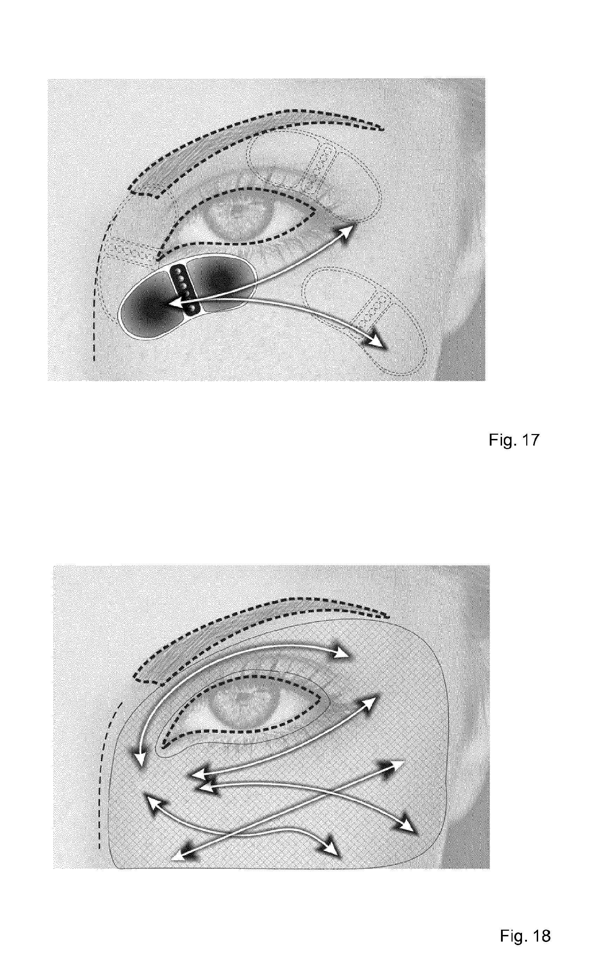

Die Fig. 17 und 18 zeigen schematische Darstellungen zum Auflegen des Applikators 9 im Bereich einer Augenpartie.

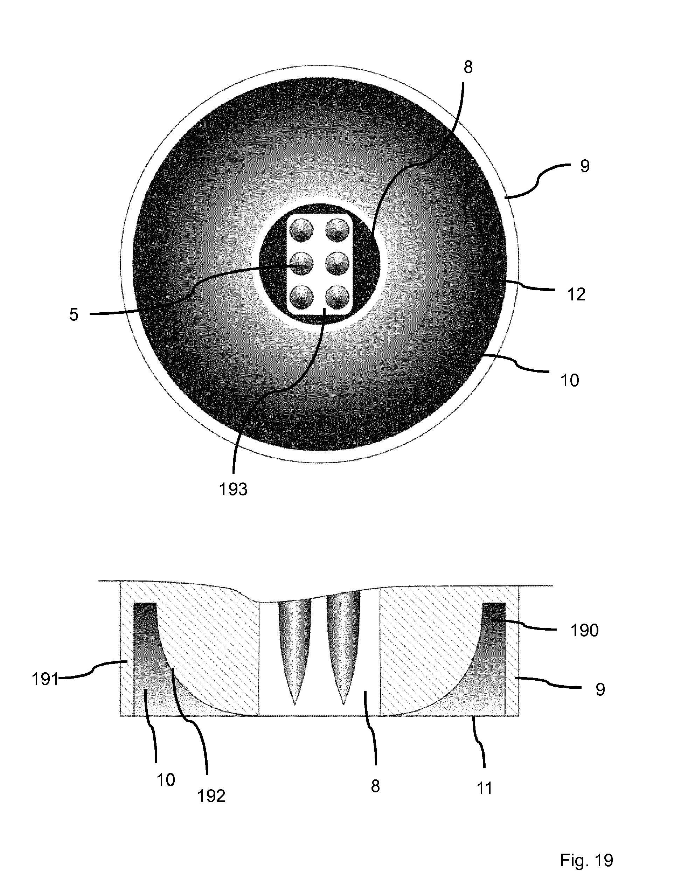

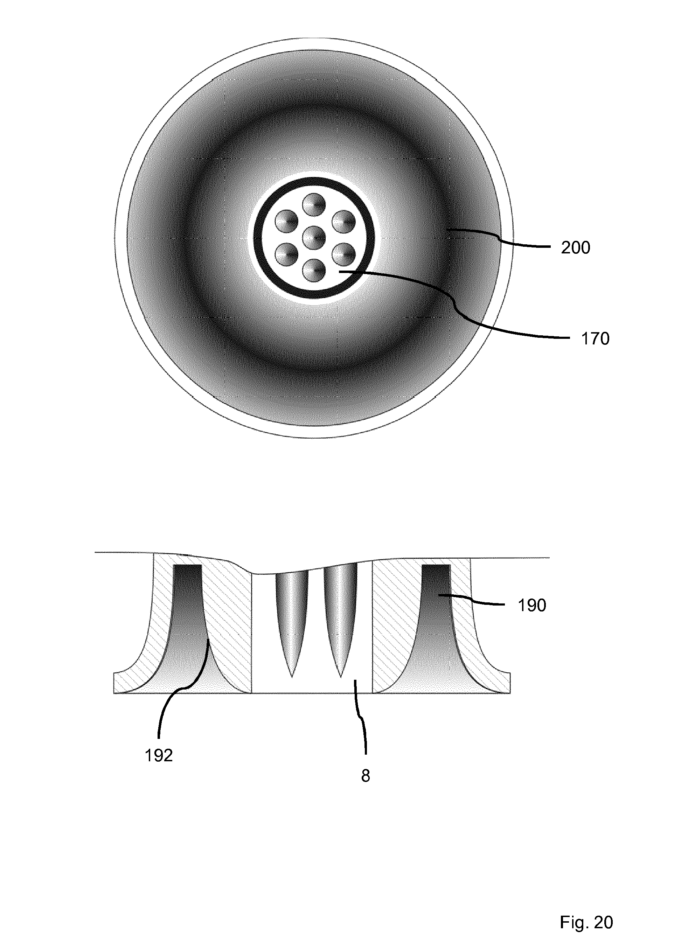

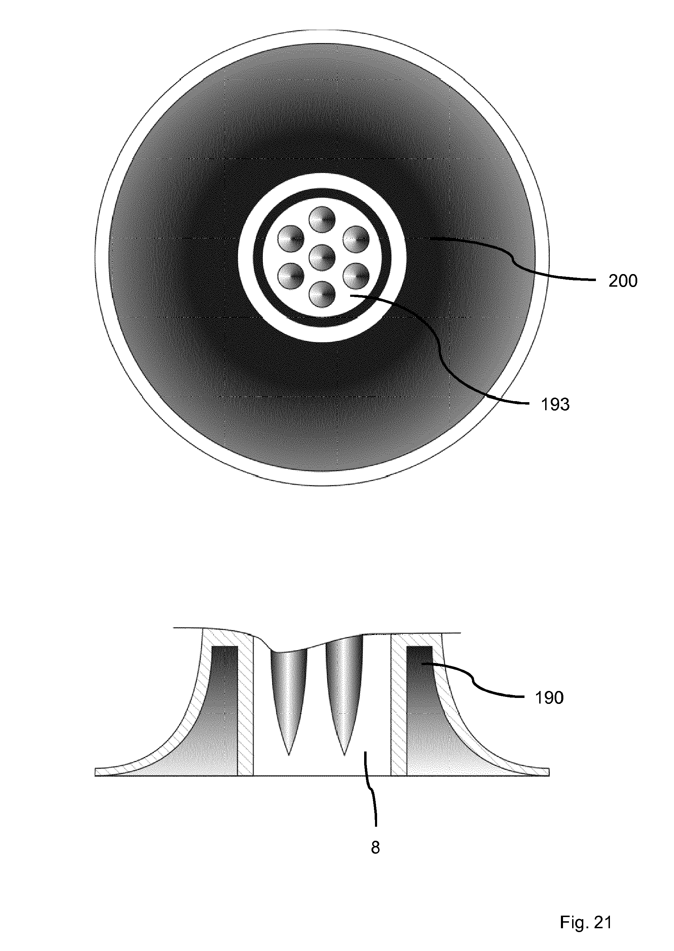

Die Fig. 19 bis 21 zeigen jeweils Ausführungsformen des Applikators 9 in Draufsicht von unten (obere Darstellung) und im Querschnitt (untere Darstellung). Insbesondere den Querschnittsdarstellungen im unteren Teil der jeweiligen Figur ist zu entnehmen, dass die Ansaugkammer 10 gegenüberliegend der Ansaugöffnung 11 einen Verjüngungsabschnitt 190 aufweist. Ebene sowie gekrümmte Wandabschnitte 191, 192 können vorgesehen sein. Das Applikationselement 5, das in der Applikationselementkammer 8 angeordnet ist, ist bei den gezeigten Ausführungsformen mit einer Anordnung von mehreren Nadeln 193 gebildet.

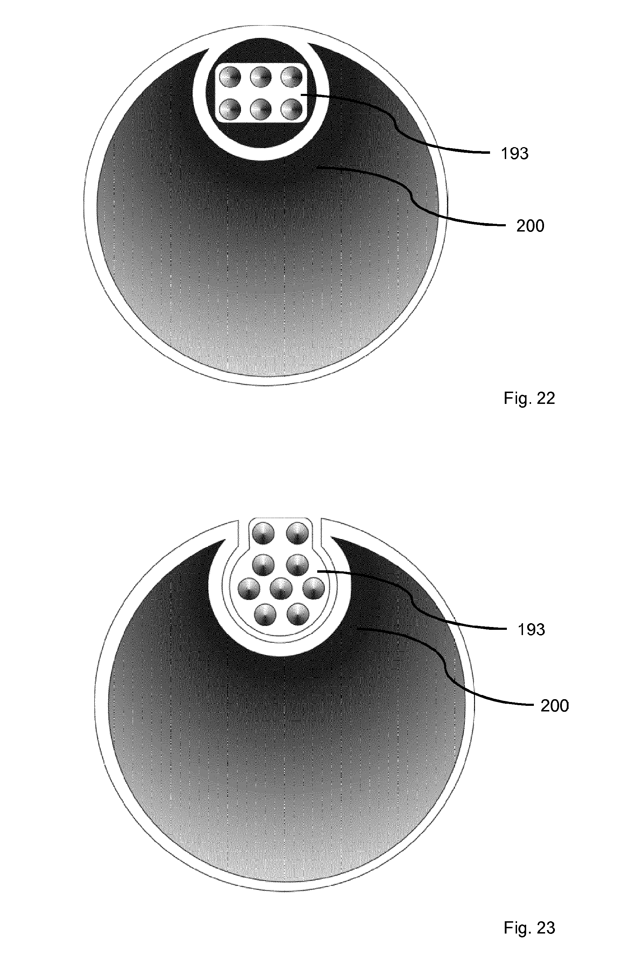

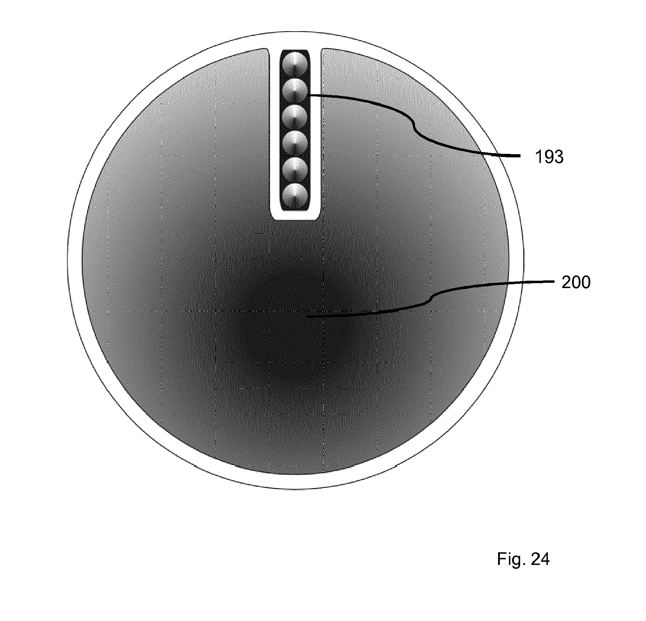

Schließlich zeigen die Fig. 22 bis 24 weitere Ausgestaltungen der Applikationsfläche 12 in Draufsicht, wobei das Applikationselement 5 jeweils als eine Nadelgruppe gebildet ist, die am seitlichen Rand der Applikationsfläche 12 angeordnet ist.

In den Fig. 1 sowie 19 bis 24 zeigen dunkler gestaltete Bereiche 200 jeweils die Lage des Verjüngungsabschnittes 190. Dieser kann außenliegend oder innenliegend angeordnet sein. Er kann die Applikationselementkammer 8 ganz oder teilweise umgreifen mit Blickrichtung auf die Applikationsfläche 12.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Applikationsmodul für ein Handgerät zum wiederholten Applizieren eines Applikationselementes auf eine menschliche oder eine tierische Haut, mit
- einem Gehäuse,
- einer Applikationseinrichtung, die in dem Gehäuse angeordnet ist und eine an einer Applikationselementaufnahme angeordnetes Applikationselement aufweist, wobei die Applikationselementaufnahme mit dem Applikationselement zwischen einer ausgefahrenen und einer eingefahrenen Stellung verlagerbar ist,
- einer Kopplungseinrichtung, die eingerichtet ist, funktionell an ein Antriebsmodul zu koppeln, so dass eine vom Antriebsmodul bereitgestellte Antriebskraft abgreifbar und zumindest für einen Antrieb der Applikationsrichtung bereitgestellt ist,
- einem an dem Gehäuse gebildeten Applikator, der im Betrieb in einem zu behandelnden Hautbereich zur Auflage kommt,
- einer Applikationselementkammer, in welcher sich das Applikationselement im Betrieb vor und zurückbewegt und die eine einem distalen Ende des Applikationselements zugeordnete Applikationselementöffnung aufweist, welche die Applikationselementkammer zum Applikator hin öffnet oder im Bereich des Applikators angeordnet ist, und
- einer Ansaugkammer, die getrennt von der Applikationselementkammer gebildet ist und einen Ansauganschluss aufweist, über den eine pneumatische Saugkraft in die Ansaugkammer einleitbar ist, wobei eine Ansaugöffnung vorgesehen ist, über die sich die Ansaugkammer zum Applikator hin öffnet oder die im Bereich des Applikators angeordnet ist.

2. Applikationsmodul nach Anspruch 1, **dadurch gekennzeichnet, dass** die Applikationselementöffnung und die Ansaugöffnung benachbart zueinander im Bereich einer Applikationsfläche angeordnet sind, die an einem distalen Ende des Applikators gebildet ist.

3. Applikationsmodul nach Anspruch 1 oder 2, **dadurch gekennzeichnet, dass** die Ansaugkammer wenigstens zum Teil von einem trompetenförmigen Wandabschnitt umgeben ist, welcher sich wenigstens teilweise auf einer Rückseite von der Ansaugöffnung weg erstreckt.

4. Applikationsmodul nach mindestens einem der vorangehenden Ansprüche, **dadurch gekennzeichnet, dass** sich die Ansaugkammer in einem der Ansaugöffnung gegenüberliegenden Kammerabschnitt im Querschnitt verjüngt.

5. Applikationsmodul nach mindestens einem der vorangehenden Ansprüche, **dadurch gekennzeichnet, dass** die Ansaugöffnung wenigstens teilweise von Wandabschnitten umgeben ist, die aus einem flexiblen Material bestehen.

6. Applikationsmodul nach mindestens einem der vorangehenden Ansprüche, **dadurch gekennzeichnet, dass** die Applikationselementkammer eine seitliche Öffnung aufweist.

7. Applikationsmodul nach mindestens einem der vorangehenden Ansprüche, **dadurch gekennzeichnet, dass** die Ansaugkammer die Applikationselementkammer mehrseitig umgibt.

8. Applikationsmodul nach mindestens einem der vorangehenden Ansprüche, **dadurch gekennzeichnet, dass** die Kopplungseinrichtung eingerichtet ist, eine mechanische, eine elektrische, eine magnetische und / oder eine pneumatische Antriebskraft zumindest für einen Antrieb der Applikationseinrichtung bereitzustellen.

9. Applikationsmodul nach mindestens einem der vorangehenden Ansprüche, **dadurch gekennzeichnet, dass** die Kopplungseinrichtung eingerichtet ist, eine drehende und / oder eine geradlinige Antriebsbewegung abzugreifen.

10. Applikationsmodul nach mindestens einem der vorangehenden Ansprüche, **dadurch gekennzeichnet, dass** die Kopplungseinrichtung eingerichtet ist, die vom Antriebsmodul bereitgestellte Antriebskraft kontaktlos abzugreifen.

11. Applikationsmodul nach mindestens einem der vorangehenden Ansprüche, dadurch **g e - kennzeichnet,** dass die Kopplungseinrichtung mit einem Wandlungsmechanismus verbunden ist, welcher im Gehäuse angeordnet und eingerichtet ist, eine Antriebsbewegungsart in eine andere und auf die Applikationseinrichtung eingekoppelte Antriebsbewegungsart zu wandeln, welche von der Antriebsbewegungsart verschieden ist.

12. Applikationsmodul nach mindestens einem der vorangehenden Ansprüche, **dadurch gekennzeichnet, dass** in dem Gehäuse eine Pumpeinrichtung angeordnet ist, die mit dem Ansauganschluss der Ansaugkammer in Fluidverbindung steht, derart, dass ein von der Pumpeinrichtung erzeugter Ansaugdruck auf die Ansaugkammer gegeben werden kann.

13. Applikationsmodul nach mindestens einem der vorangehenden Ansprüche, **dadurch gekennzeichnet, dass** in dem Gehäuse ein Fluidreservoir gebildet ist.

14. Applikationsmodul nach mindestens einem der vorangehenden Ansprüche, **dadurch gekennzeichnet, dass** die Applikationseinrichtung eine Stecheinrichtung ist, die an der als Stechelementaufnahme ausgeführten Applikationselementaufnahme das als Stechelement gebildetes Applikationselement aufweist, wobei die Applikationselementaufnahme mit dem Applikationselement, wobei am distalen Ende des Stechelements eine stechspitze angeordnet ist, die eingerichtet ist, die Haut aufzustechen.

15. Handgerät zum wiederholten Applizieren eines Applikationselementes auf eine menschliche oder eine tierische Haut mit einem Antriebsmodul, welches eingerichtet ist, eine Antriebskraft bereitzustellen, und einem Applikationsmodul nach mindestens einem der vorangehenden Ansprüche, welches funktionell an das Antriebsmodul koppelt, so dass die vom Antriebsmodul bereitgestellte Antriebskraft abgreifbar und im Applikationsmodul zumindest für einen Antrieb einer Applikationseinrichtung bereitgestellt ist.
